# EUROPEAN PATENT SPECIFICATION

(11) **EP 4 110 164 B1**
(45) Date of publication and mention of the grant of the patent: **22.07.2026**
(21) Application number: 21713558.1
(22) Date of filing: 01.03.2021
(51) Int. Cl.: A61B 5/00, G16H 50/20, G16H 50/30, A61B 5/021

(54) **PREDICTION OF RISK OF PRE-ECLAMPSIA**
VORHERSAGE DES RISIKOS VON PRÄEKLAMPSIE
PRÉDICTION D'UN RISQUE DE PRÉÉCLAMPSIE

(30) Priority: 28.02.2020 GB 202002909; 27.07.2020 GB 202011592
(43) Date of publication of application: 04.01.2023
(73) Proprietor: Metabolomic Diagnostics Limited, Cork (IE)
(72) Inventor: TUYTTEN, Robin, Midleton, Co. Cork (IE)
(74) Representative: Purdylucey Intellectual Property
(86) International application number: PCT/EP2021/055085
(87) International publication number: WO 2021/170885

(56) References cited:
- US-A1- 2013 073 212
- US-A1- 2018 114 600
- ASMA KHALIL ET AL: "First trimester markers for the prediction of pre-eclampsia in women with a priori high risk", ULTRASOUND IN OBSTETRICS AND GYNECOLOGY, 1 January 2010 (2010-01-01), GB, pages n/a - n/a, XP055237632, ISSN: 0960-7692, DOI: 10.1002/uog.7559

## Description

### Field of the Invention

The present invention relates to a system for the early prediction of pre-eclampsia, especially pre-term pre-eclampsia and term pre-eclampsia.

### Background to the Invention

Preeclampsia is a syndromic condition affecting 2-8% of pregnancies worldwide [1].

Recently it has been demonstrated that preterm preeclampsia (preeclampsia leading to delivery before 37 weeks of gestation) can be prevented by prophylactic administration of aspirin in women found at risk preterm pre-eclampsia [2]. About 30% of all pre-eclampsia is preterm preeclampsia. For prevention of term preeclampsia, other interventions may be more relevant, e.g. like metformin. The latter has been shown to prevent preeclampsia in obese pregnant women [3]. An elevated blood pressure is a long-established risk factor for prediction pre-eclampsia risk. Typically, the blood measurement used is mean arterial pressure, which is calculated as map = [(2 x diastolic bp + 1 x systolic bp)/3].

Pre-eclampsia is generally recognised by clinicians to develop in pregnant women from 20 weeks gestation [13]. Prior to 20 weeks gestation the disease does not appear in pregnant women, although women may present with other hypertensive disorders prior to 20 weeks that are distinct from pre-eclampsia. As pre-eclampsia is such a dangerous and devastating condition for mother and child, there is a need to screen pregnant women early in pregnancy, before they develop pre-eclampsia, to identify women who are at risk of developing pre-eclampsia later in pregnancy. The purpose of this screening is to allow early identification of risk and consequent lifestyle changes (e.g. diet, exercise) and increased surveillance, to try and prevent development of pre-eclampsia or allow positive diagnosis of the disease as early as possible after screening due to more frequent surveillance.

Systems and methods for predicting the early onset of pre-eclampsia in pregnant women are known from documents US 2013/073212 A1, ASMA KHALIL ET AL: "First trimester markers for the prediction of pre-eclampsia in women with a priori high risk", Ultrasound Obstet Gynecol 2010; 35: 671-679, and US 2018/114600 A1.

### Summary of the Invention

The Applicant has discovered that calculating a pregnant woman's "body mass index" and combining this patient characteristic with a blood pressure measurement enables a more fine-grained prediction of preeclampsia risk prior to 20 weeks of pregnancy, at a time when the disease is not present, allowing the woman to be monitored more carefully and also to initiate lifestyle changes which may prevent the development of the disease. Early screening for risk also specifically allows to differentiate the risks for developing preterm and term preeclampsia at an early stage of pregnancy, which may inform for different intervention strategies during the second and third trimesters of pregnancy. Thus, the invention relates to a system for screening a woman early in pregnancy to determine risk of the woman subsequently developing pre-eclampsia later in pregnancy. The screening is typically performed at 11-19 weeks pregnancy, which is not only before the clinical symptoms of pre-eclampsia appear, but also before the disease manifests itself in a pregnant woman.

The invention is defined by independent claim 1. Preferred embodiments are defined by dependent claims 2-5.

### Brief Description of the Figures

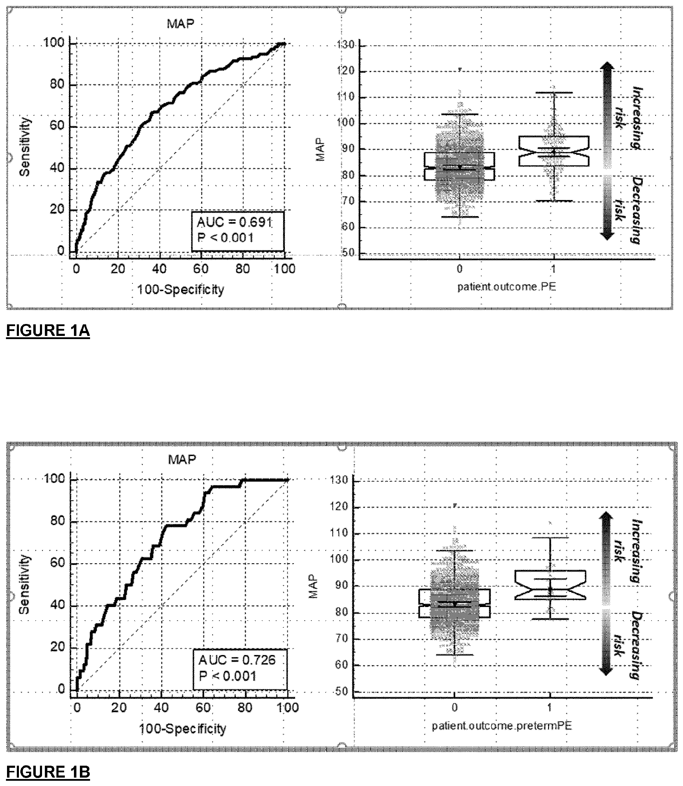

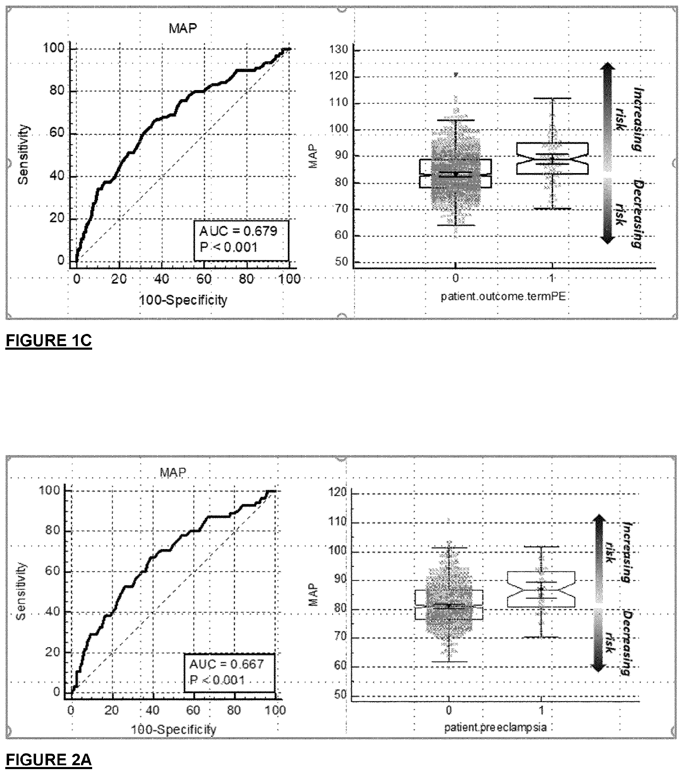

**Figure 1****:** AU-ROC and box plots for cases and controls for all pregnant women
   *Prediction of All PE (****Figure 1A****)*
   Receiver operating curve for MAP - If P is small (P<0.05) then it can be concluded that the Area under the ROC curve is significantly different from 0.5 and that therefore MAP has the ability to distinguish between women who will develop PE and women who will not develop PE later in their pregnancy.
   Notched box and whisker graphs comparing the distribution of MAP in pregnant women not developing PE (0) and women developing PE (1) later in their pregnancies. The central box represents the values from the lower to upper quartile (25-75 percentile). The middle line represents the median (in-box whiskers: 95% CI for median). Whiskers: lower quartile minus 1.5 times the interquartile range, or larger than the upper quartile plus 1.5 times the interquartile range (inner fences).
   *Prediction of Preterm PE (****Figure 1B****)*
   Receiver operating curve for MAP - If P is small (P<0.05) then it can be concluded that the Area under the ROC curve is significantly different from 0.5 and that therefore MAP has the ability to distinguish between women who will develop preterm PE and women who will not develop PE later in their pregnancy
   Notched box and whisker graphs comparing the distribution of MAP in pregnant women not developing PE (0) and women developing preterm PE (1) later in their pregnancies. The central box represents the values from the lower to upper quartile (25-75 percentile). The middle line represents the median (in-box whiskers: 95% CI for median). Whiskers: lower quartile minus 1.5 times the interquartile range, or larger than the upper quartile plus 1.5 times the interquartile range (inner fences).
   *Prediction of Term PE (****Figure 1C****)*
   Receiver operating curve for MAP - If P is small (P<0.05) then it can be concluded that the Area under the ROC curve is significantly different from 0.5 and that therefore MAP has the ability to distinguish between women who will develop term PE and women who will not develop PE later in their pregnancy
   Notched box and whisker graphs comparing the distribution of MAP in pregnant women not developing PE (0) and women developing term PE (1) later in their pregnancies. The central box represents the values from the lower to upper quartile (25-75 percentile). The middle line represents the median (in-box whiskers: 95% CI for median). Whiskers: lower quartile minus 1.5 times the interquartile range, or larger than the upper quartile plus 1.5 times the interquartile range (inner fences).

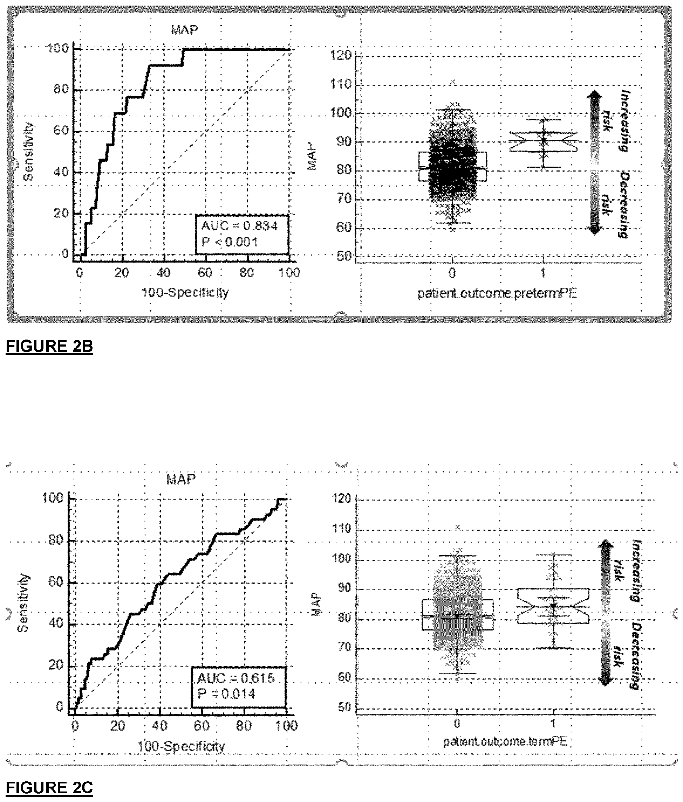

**Figure 2**: AU-ROC and box plots for cases and controls for low BMI pregnant women
   *Prediction of All PE (****Figure 2A****)*
   Receiver operating curve for MAP - If P is small (P<0.05) then it can be concluded that the Area under the ROC curve is significantly different from 0.5 and that therefore MAP has the ability to distinguish between women who will develop PE and women who will not develop PE later in their pregnancy
   Notched box and whisker graphs comparing the distribution of MAP in pregnant women not developing PE (0) and women developing PE (1) later in their pregnancies. The central box represents the values from the lower to upper quartile (25-75 percentile). The middle line represents the median (in-box whiskers: 95% CI for median). Whiskers: lower quartile minus 1.5 times the interquartile range, or larger than the upper quartile plus 1.5 times the interquartile range (inner fences).
   *Prediction of Preterm PE (****Figure 2B****)*
   Receiver operating curve for MAP - If P is small (P<0.05) then it can be concluded that the Area under the ROC curve is significantly different from 0.5 and that therefore MAP has the ability to distinguish between women who will develop preterm PE and women who will not develop PE later in their pregnancy
   Notched box and whisker graphs comparing the distribution of MAP in pregnant women not developing PE (0) and women developing preterm PE (1) later in their pregnancies. The central box represents the values from the lower to upper quartile (25-75 percentile). The middle line represents the median (in-box whiskers: 95% CI for median). Whiskers: lower quartile minus 1.5 times the interquartile range, or larger than the upper quartile plus 1.5 times the interquartile range (inner fences).
   *Prediction of Term PE (****Figure 2C****)*
   Receiver operating curve for MAP - If P is small (P<0.05) then it can be concluded that the Area under the ROC curve is significantly different from 0.5 and that therefore MAP has the ability to distinguish between women who will develop term PE and women who will not develop PE later in their pregnancy
   Notched box and whisker graphs comparing the distribution of MAP in pregnant women not developing PE (0) and women developing term PE (1) later in their pregnancies. The central box represents the values from the lower to upper quartile (25-75 percentile). The middle line represents the median (in-box whiskers: 95% CI for median). Whiskers: lower quartile minus 1.5 times the interquartile range, or larger than the upper quartile plus 1.5 times the interquartile range (inner fences).

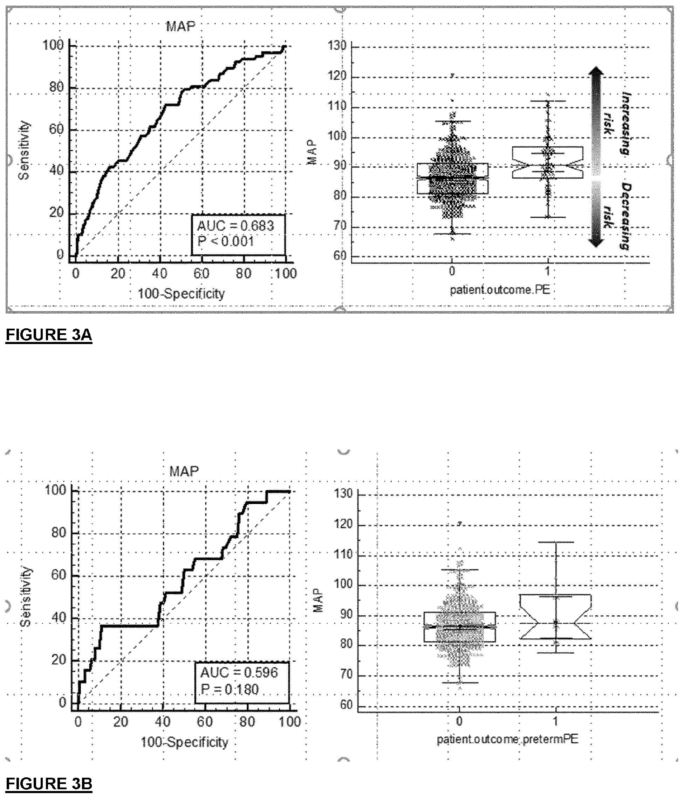

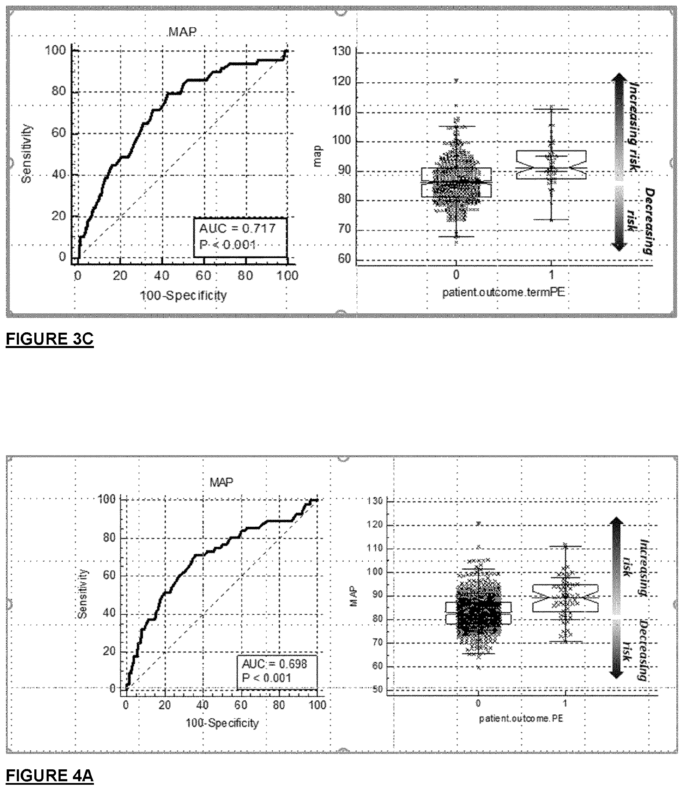

**Figure 3****:** AU-ROC and box plots for cases and controls for high BMI pregnant women
   *Prediction of All PE (****Figure 3A****)*
   Receiver operating curve for MAP - If P is small (P<0.05) then it can be concluded that the Area under the ROC curve is significantly different from 0.5 and that therefore MAP has the ability to distinguish between women who will develop PE and women who will not develop PE later in their pregnancy
   Notched box and whisker graphs comparing the distribution of MAP in pregnant women not developing PE (0) and women developing PE (1) later in their pregnancies. The central box represents the values from the lower to upper quartile (25-75 percentile). The middle line represents the median (in-box whiskers: 95% CI for median). Whiskers: lower quartile minus 1.5 times the interquartile range, or larger than the upper quartile plus 1.5 times the interquartile range (inner fences).
   *Prediction of Preterm PE (****Figure 3B****)*
   Receiver operating curve for MAP - If P is small (P<0.05) then it can be concluded that the Area under the ROC curve is significantly different from 0.5 and that therefore MAP has the ability to distinguish between women who will develop preterm PE and women who will not develop PE later in their pregnancy
   Notched box and whisker graphs comparing the distribution of MAP in pregnant women not developing PE (0) and women developing preterm PE (1) later in their pregnancies. The central box represents the values from the lower to upper quartile (25-75 percentile). The middle line represents the median (in-box whiskers: 95% CI for median). Whiskers: lower quartile minus 1.5 times the interquartile range, or larger than the upper quartile plus 1.5 times the interquartile range (inner fences).
   *Prediction of Term PE (****Figure 3C****)*
   Receiver operating curve for MAP - If P is small (P<0.05) then it can be concluded that the Area under the ROC curve is significantly different from 0.5 and that therefore MAP has the ability to distinguish between women who will develop term PE and women who will not develop PE later in their pregnancy
   Notched box and whisker graphs comparing the distribution of MAP in pregnant women not developing PE (0) and women developing term PE (1) later in their pregnancies. The central box represents the values from the lower to upper quartile (25-75 percentile). The middle line represents the median (in-box whiskers: 95% CI for median). Whiskers: lower quartile minus 1.5 times the interquartile range, or larger than the upper quartile plus 1.5 times the interquartile range (inner fences).

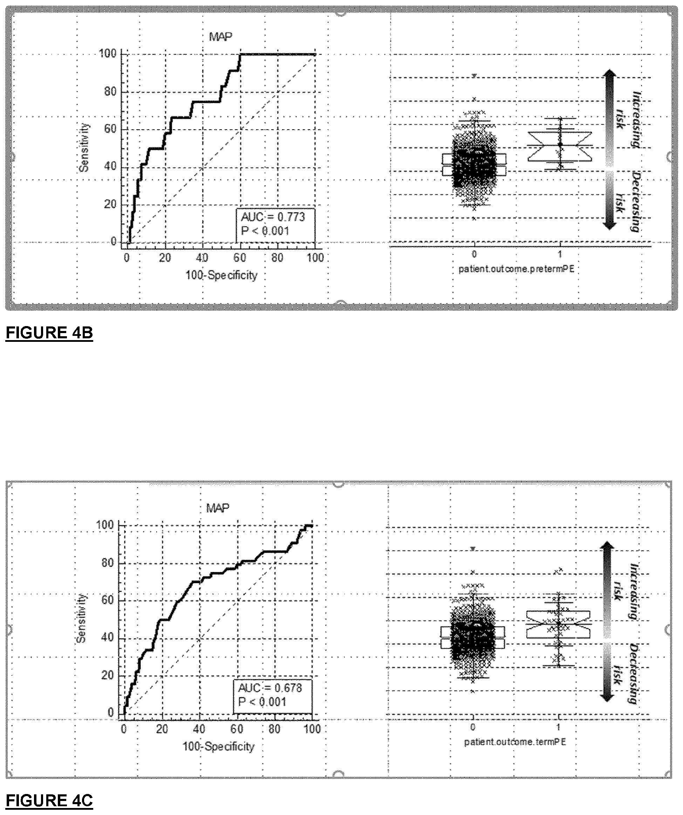

**Figure 4****:** AU-ROC and box plots for cases and controls for pregnant women carrying a female fetus
   *Prediction of All PE (****Figure 4A****)*
   Receiver operating curve for MAP - If P is small (P<0.05) then it can be concluded that the Area under the ROC curve is significantly different from 0.5 and that therefore MAP has the ability to distinguish between women who will develop PE and women who will not develop PE later in their pregnancy
   Notched box and whisker graphs comparing the distribution of MAP in pregnant women not developing PE (0) and women developing PE (1) later in their pregnancies. The central box represents the values from the lower to upper quartile (25-75 percentile). The middle line represents the median (in-box whiskers: 95% CI for median). Whiskers: lower quartile minus 1.5 times the interquartile range, or larger than the upper quartile plus 1.5 times the interquartile range (inner fences).
   *Prediction of Preterm PE (****Figure 4B****)*
   Receiver operating curve for MAP - If P is small (P<0.05) then it can be concluded that the Area under the ROC curve is significantly different from 0.5 and that therefore MAP has the ability to distinguish between women who will develop preterm PE and women who will not develop PE later in their pregnancy
   Notched box and whisker graphs comparing the distribution of MAP in pregnant women not developing PE (0) and women developing preterm PE (1) later in their pregnancies. The central box represents the values from the lower to upper quartile (25-75 percentile). The middle line represents the median (in-box whiskers: 95% CI for median). Whiskers: lower quartile minus 1.5 times the interquartile range, or larger than the upper quartile plus 1.5 times the interquartile range (inner fences).
   *Prediction of Term PE (****Figure 4C****)*
   Receiver operating curve for MAP - If P is small (P<0.05) then it can be concluded that the Area under the ROC curve is significantly different from 0.5 and that therefore MAP has the ability to distinguish between women who will develop term PE and women who will not develop PE later in their pregnancy
   Notched box and whisker graphs comparing the distribution of MAP in pregnant women not developing PE (0) and women developing term PE (1) later in their pregnancies. The central box represents the values from the lower to upper quartile (25-75 percentile). The middle line represents the median (in-box whiskers: 95% CI for median). Whiskers: lower quartile minus 1.5 times the interquartile range, or larger than the upper quartile plus 1.5 times the interquartile range (inner fences).

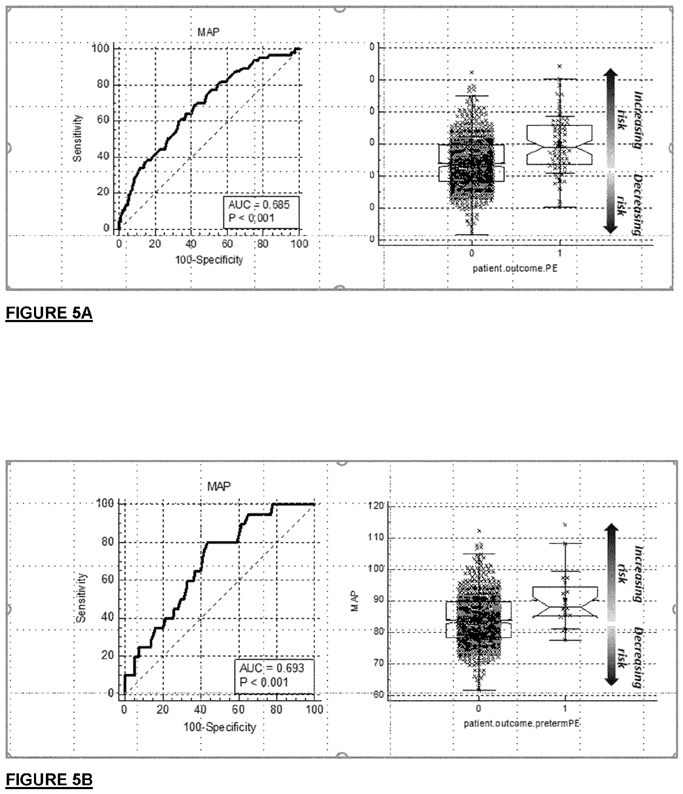

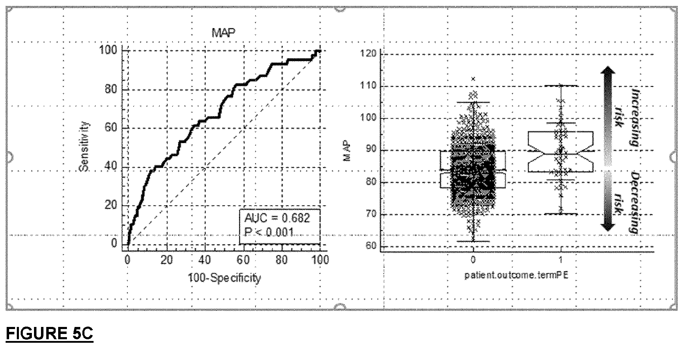

**Figure 5****:** AU-ROC and box plots for cases and controls for pregnant women carrying a male fetus
   Prediction of All PE (**Figure 5A**)
   Receiver operating curve for MAP - If P is small (P<0.05) then it can be concluded that the Area under the ROC curve is significantly different from 0.5 and that therefore MAP has the ability to distinguish between women who will develop PE and women who will not develop PE later in their pregnancy
   Notched box and whisker graphs comparing the distribution of MAP in pregnant women not developing PE (0) and women developing PE (1) later in their pregnancies. The central box represents the values from the lower to upper quartile (25-75 percentile). The middle line represents the median (in-box whiskers: 95% CI for median). Whiskers: lower quartile minus 1.5 times the interquartile range, or larger than the upper quartile plus 1.5 times the interquartile range (inner fences).
   Prediction of Preterm PE (**Figure 5B**)
   Receiver operating curve for MAP - If P is small (P<0.05) then it can be concluded that the Area under the ROC curve is significantly different from 0.5 and that therefore MAP has the ability to distinguish between women who will develop preterm PE and women who will not develop PE later in their pregnancy
   Notched box and whisker graphs comparing the distribution of MAP in pregnant women not developing PE (0) and women developing preterm PE (1) later in their pregnancies. The central box represents the values from the lower to upper quartile (25-75 percentile). The middle line represents the median (in-box whiskers: 95% CI for median). Whiskers: lower quartile minus 1.5 times the interquartile range, or larger than the upper quartile plus 1.5 times the interquartile range (inner fences).
   Prediction of Term PE (**Figure 5C**)
   Receiver operating curve for MAP - If P is small (P<0.05) then it can be concluded that the Area under the ROC curve is significantly different from 0.5 and that therefore MAP has the ability to distinguish between women who will develop term PE and women who will not develop PE later in their pregnancy
   Notched box and whisker graphs comparing the distribution of MAP in pregnant women not developing PE (0) and women developing term PE (1) later in their pregnancies. The central box represents the values from the lower to upper quartile (25-75 percentile). The middle line represents the median (in-box whiskers: 95% CI for median). Whiskers: lower quartile minus 1.5 times the interquartile range, or larger than the upper quartile plus 1.5 times the interquartile range (inner fences).

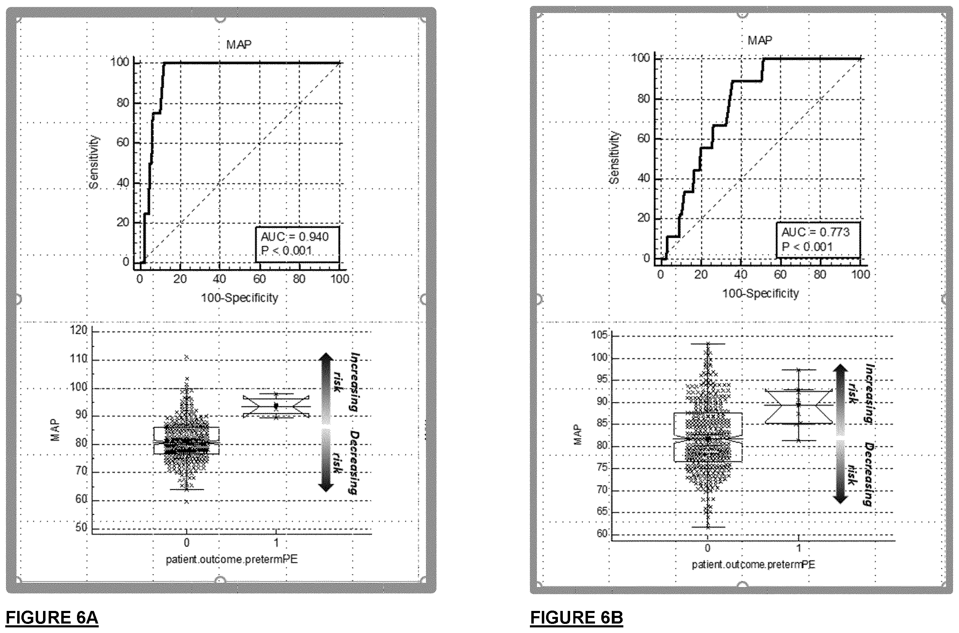

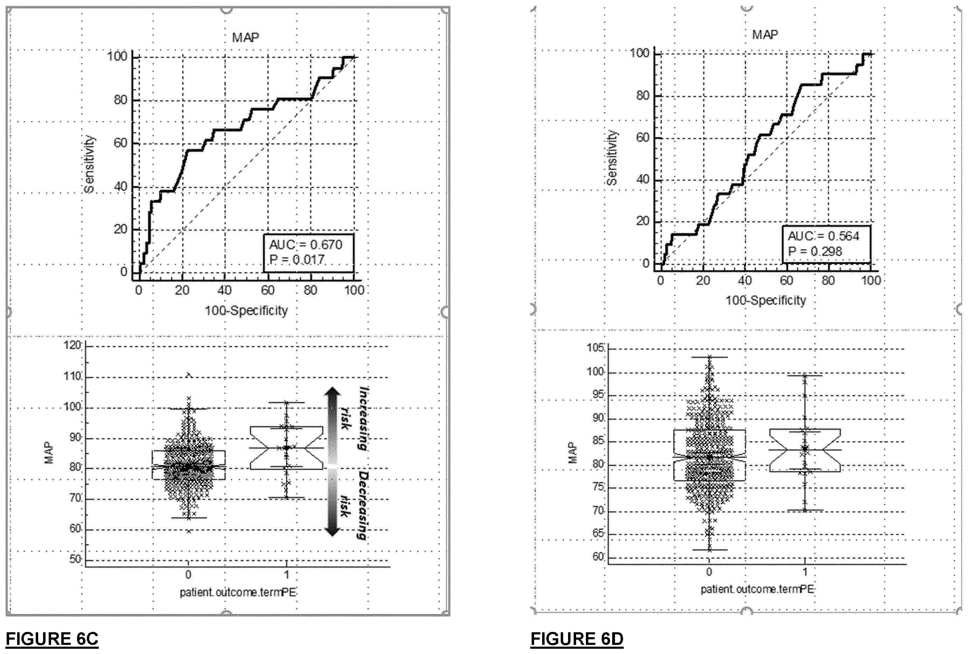

**Figure 6**: AU-ROC and box plots for cases and controls for low BMI pregnant women further stratified according to fetal sex
   Prediction of Preterm PE in women carrying a female fetus (**Figure 6A**)
   Receiver operating curve for MAP - If P is small (P<0.05) then it can be concluded that the Area under the ROC curve is significantly different from 0.5 and that therefore MAP has the ability to distinguish between women who will develop preterm PE and women who will not develop PE later in their pregnancy
   Notched box and whisker graphs comparing the distribution of MAP in pregnant women not developing PE (0) and women developing preterm PE (1) later in their pregnancies. The central box represents the values from the lower to upper quartile (25-75 percentile). The middle line represents the median (in-box whiskers: 95% CI for median). Whiskers: lower quartile minus 1.5 times the interquartile range, or larger than the upper quartile plus 1.5 times the interquartile range (inner fences).
   Prediction of Preterm PE in women carrying a male fetus (**Figure 6B**)
   Receiver operating curve for MAP - If P is small (P<0.05) then it can be concluded that the Area under the ROC curve is significantly different from 0.5 and that therefore MAP has the ability to distinguish between women who will develop preterm PE and women who will not develop PE later in their pregnancy
   Notched box and whisker graphs comparing the distribution of MAP in pregnant women not developing PE (0) and women developing preterm PE (1) later in their pregnancies. The central box represents the values from the lower to upper quartile (25-75 percentile). The middle line represents the median (in-box whiskers: 95% CI for median). Whiskers: lower quartile minus 1.5 times the interquartile range, or larger than the upper quartile plus 1.5 times the interquartile range (inner fences).
   Prediction of Term PE in women carrying a female fetus (**Figure 6C**)
   Receiver operating curve for MAP - If P is small (P<0.05) then it can be concluded that the Area under the ROC curve is significantly different from 0.5 and that therefore MAP has the ability to distinguish between women who will develop term PE and women who will not develop PE later in their pregnancy
   Notched box and whisker graphs comparing the distribution of MAP in pregnant women not developing PE (0) and women developing term PE (1) later in their pregnancies. The central box represents the values from the lower to upper quartile (25-75 percentile). The middle line represents the median (in-box whiskers: 95% CI for median). Whiskers: lower quartile minus 1.5 times the interquartile range, or larger than the upper quartile plus 1.5 times the interquartile range (inner fences).
   Prediction of Term PE in women carrying a male fetus (**Figure 6D**)
   Receiver operating curve for MAP - If P is small (P<0.05) then it can be concluded that the Area under the ROC curve is significantly different from 0.5 and that therefore MAP has the ability to distinguish between women who will develop term PE and women who will not develop PE later in their pregnancy
   Notched box and whisker graphs comparing the distribution of MAP in pregnant women not developing PE (0) and women developing term PE (1) later in their pregnancies. The central box represents the values from the lower to upper quartile (25-75 percentile). The middle line represents the median (in-box whiskers: 95% CI for median). Whiskers: lower quartile minus 1.5 times the interquartile range, or larger than the upper quartile plus 1.5 times the interquartile range (inner fences).

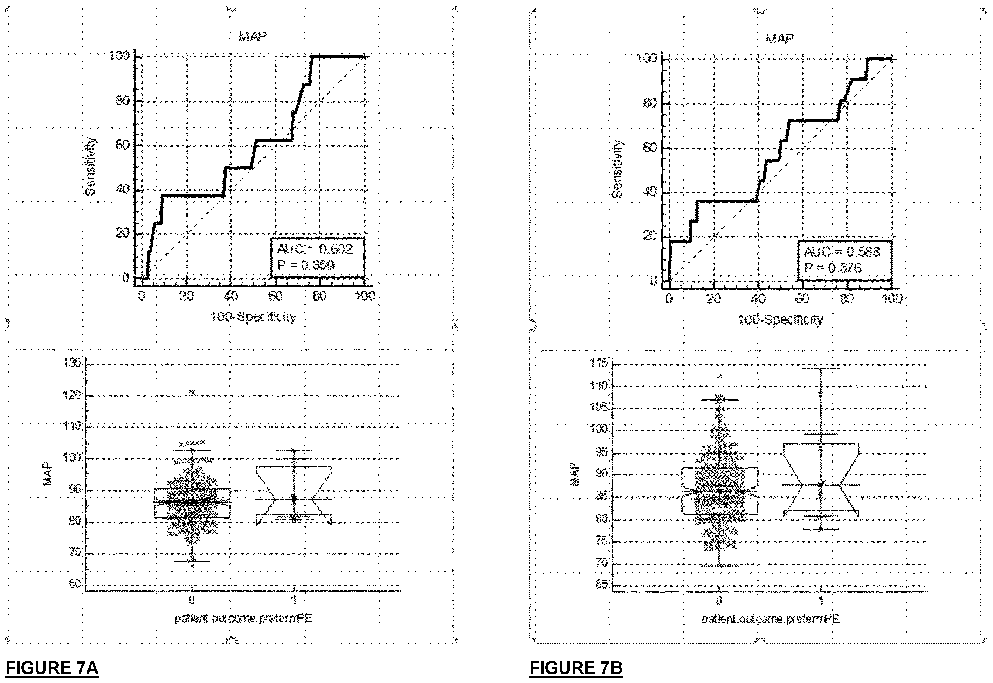

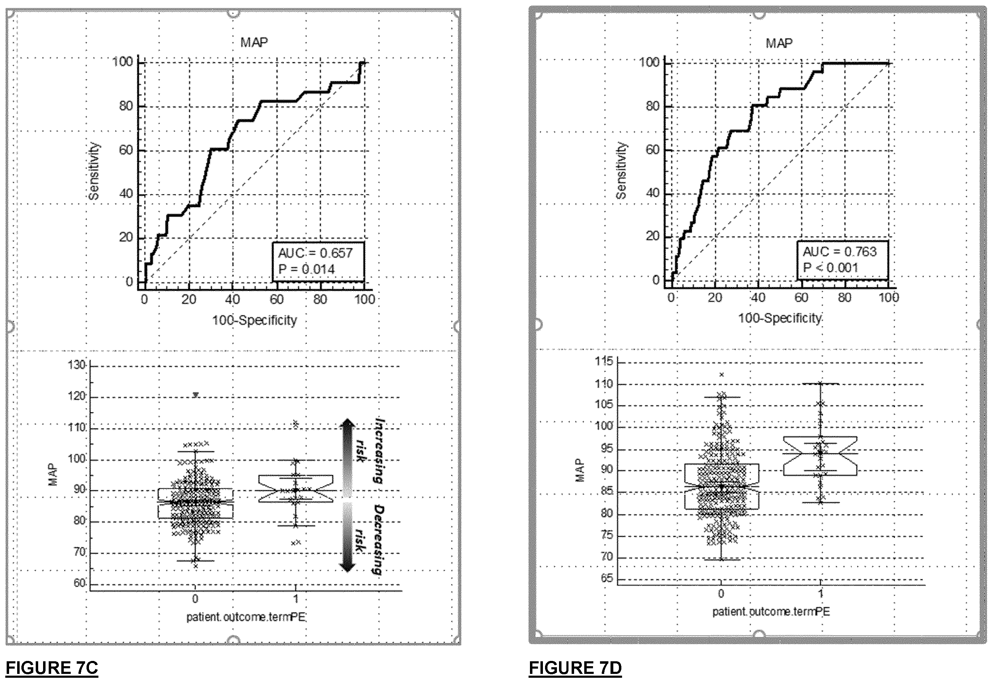

Figure 7: AU-ROC and box plots for cases and controls for high BMI pregnant women further stratified according to fetal sex
   Prediction of Preterm PE in women carrying a female fetus (**Figure 7A**)
   Receiver operating curve for MAP - If P is small (P<0.05) then it can be concluded that the Area under the ROC curve is significantly different from 0.5 and that therefore MAP has the ability to distinguish between women who will develop preterm PE and women who will not develop PE later in their pregnancy
   Notched box and whisker graphs comparing the distribution of MAP in pregnant women not developing PE (0) and women developing preterm PE (1) later in their pregnancies. The central box represents the values from the lower to upper quartile (25-75 percentile). The middle line represents the median (in-box whiskers: 95% CI for median). Whiskers: lower quartile minus 1.5 times the interquartile range, or larger than the upper quartile plus 1.5 times the interquartile range (inner fences).
   Prediction of Preterm PE in women carrying a male fetus (**Figure 7B**)
   Receiver operating curve for MAP - If P is small (P<0.05) then it can be concluded that the Area under the ROC curve is significantly different from 0.5 and that therefore MAP has the ability to distinguish between women who will develop preterm PE and women who will not develop PE later in their pregnancy
   Notched box and whisker graphs comparing the distribution of MAP in pregnant women not developing PE (0) and women developing preterm PE (1) later in their pregnancies. The central box represents the values from the lower to upper quartile (25-75 percentile). The middle line represents the median (in-box whiskers: 95% CI for median). Whiskers: lower quartile minus 1.5 times the interquartile range, or larger than the upper quartile plus 1.5 times the interquartile range (inner fences).
   Prediction of Term PE in women carrying a female fetus (**Figure 7C**)
   Receiver operating curve for MAP - If P is small (P<0.05) then it can be concluded that the Area under the ROC curve is significantly different from 0.5 and that therefore MAP has the ability to distinguish between women who will develop term PE and women who will not develop PE later in their pregnancy
   Notched box and whisker graphs comparing the distribution of MAP in pregnant women not developing PE (0) and women developing term PE (1) later in their pregnancies. The central box represents the values from the lower to upper quartile (25-75 percentile). The middle line represents the median (in-box whiskers: 95% CI for median). Whiskers: lower quartile minus 1.5 times the interquartile range, or larger than the upper quartile plus 1.5 times the interquartile range (inner fences).
   Prediction of Term PE in women carrying a male fetus (**Figure 7D**)
   Receiver operating curve for MAP - If P is small (P<0.05) then it can be concluded that the Area under the ROC curve is significantly different from 0.5 and that therefore MAP has the ability to distinguish between women who will develop term PE and women who will not develop PE later in their pregnancy
   Notched box and whisker graphs comparing the distribution of MAP in pregnant women not developing PE (0) and women developing term PE (1) later in their pregnancies. The central box represents the values from the lower to upper quartile (25-75 percentile). The middle line represents the median (in-box whiskers: 95% CI for median). Whiskers: lower quartile minus 1.5 times the interquartile range, or larger than the upper quartile plus 1.5 times the interquartile range (inner fences).

### Detailed Description of the Invention

### Definitions and general preferences

Where used herein and unless specifically indicated otherwise, the following terms are intended to have the following meanings in addition to any broader (or narrower) meanings the terms might enjoy in the art:
Unless otherwise required by context, the use herein of the singular is to be read to include the plural and *vice versa.* The term "a" or "an" used in relation to an entity is to be read to refer to one or more of that entity. As such, the terms "a" (or "an"), "one or more," and "at least one" are used interchangeably herein.

As used herein, the term "comprise," or variations thereof such as "comprises" or "comprising," are to be read to indicate the inclusion of any recited integer (e.g. a feature, element, characteristic, property, method/process step or limitation) or group of integers (e.g. features, element, characteristics, properties, method/process steps or limitations) but not the exclusion of any other integer or group of integers. Thus, as used herein the term "comprising" is inclusive or open-ended and does not exclude additional, unrecited integers or method/process steps.

As used herein, the term "disease" is used to define any abnormal condition that impairs physiological function and is associated with specific symptoms. The term is used broadly to encompass any disorder, illness, abnormality, pathology, sickness, condition or syndrome in which physiological function is impaired irrespective of the nature of the aetiology (or indeed whether the aetiological basis for the disease is established). It therefore encompasses conditions arising from infection, trauma, injury, surgery, radiological ablation, poisoning or nutritional deficiencies.

As used herein, the term "treatment" or "treating" refers to an intervention (e.g. the administration of an agent to a subject) which cures, ameliorates or lessens the symptoms of a disease or removes (or lessens the impact of) its cause(s) (for example, the reduction in accumulation of pathological levels of lysosomal enzymes). In this case, the term is used synonymously with the term "therapy".

Additionally, the terms "treatment" or "treating" refers to an intervention (e.g. the administration of an agent to a subject) which prevents or delays the onset or progression of a disease or reduces (or eradicates) its incidence within a treated population. In this case, the term treatment is used synonymously with the term "prophylaxis".

As used herein, an effective amount or a therapeutically effective amount of an agent defines an amount that can be administered to a subject without excessive toxicity, irritation, allergic response, or other problem or complication, commensurate with a reasonable benefit/risk ratio, but one that is sufficient to provide the desired effect, e.g. the treatment or prophylaxis manifested by a permanent or temporary improvement in the subject's condition. The amount will vary from subject to subject, depending on the age and general condition of the individual, mode of administration and other factors. Thus, while it is not possible to specify an exact effective amount, those skilled in the art will be able to determine an appropriate "effective" amount in any individual case using routine experimentation and background general knowledge. A therapeutic result in this context includes eradication or lessening of symptoms, reduced pain or discomfort, prolonged survival, improved mobility and other markers of clinical improvement. A therapeutic result need not be a complete cure.

In the context of treatment and effective amounts as defined above, the term subject (which is to be read to include "individual", "animal", "patient" or "mammal" where context permits) defines any subject, particularly a mammalian subject, for whom treatment is indicated. Mammalian subjects include, but are not limited to, humans, domestic animals, farm animals, zoo animals, sport animals, pet animals such as dogs, cats, guinea pigs, rabbits, rats, mice, horses, cattle, cows; primates such as apes, monkeys, orangutans, and chimpanzees; canids such as dogs and wolves; felids such as cats, lions, and tigers; equids such as horses, donkeys, and zebras; food animals such as cows, pigs, and sheep; ungulates such as deer and giraffes; and rodents such as mice, rats, hamsters and guinea pigs. In preferred embodiments, the subject is a human.

Unless otherwise required by context, the use of the terms "AUROC" and "AUC" are used interchangeably herein.

As used herein, the term "at risk of developing pre-term pre-eclampsia" should be understood to mean a risk that is higher than the general population (or sub-population) of pregnant woman. Likewise, the term "at risk of developing term pre-eclampsia" should be understood to mean a risk that is higher than the general population (or sub-population) of pregnant woman. For a pregnant woman that is identified by the method or system of the invention as being at risk of term or pre-term PE, the level of risk increases proportionally with the elevation in blood pressure. Thus, the more the woman's blood pressure is elevated compared with the reference blood pressure, the greater the risk. Likewise, for a pregnant woman that is identified by the method or system of the invention as being not at risk of term or pre-term PE, the reduction of risk decreases proportionally with the reduction in blood pressure.

In one embodiment, the term implies an AUROC predictive performance of at least 0.65. In one embodiment, the term implies an AUROC predictive performance of at least 0.70. In one embodiment, the term implies an AUROC predictive performance of at least 0.75. In one embodiment, the term implies an AUROC predictive performance of at least 0.80. In one embodiment, the term implies an AUROC predictive performance of at least 0.85. In one embodiment, the term implies an AUROC predictive performance of at least 0.90.

As used herein, the term "early detection of risk of pre-eclampsia" means detection of risk prior to the appearance of symptoms of the syndrome, for example during the second trimester of pregnancy (or late first trimester or early third trimester), for example from 8 to 24 weeks, or from 10 and 22 weeks, and ideally about 16 weeks (+/- 2 or 3 weeks) gestation. The term "early stage of pregnancy" means prior to the appearance of clinical symptoms of the syndrome, for example during the second trimester of pregnancy, for example from 8 to 24 weeks or from 10 to 22 weeks or from 11 to 19 weeks, and ideally about 16 weeks (+/-2, 3, 4 or 5 weeks).

A pregnant woman is diagnosed with preeclampsia when the woman has gestational hypertension (systolic BP ≥ 140 mmHg and/or diastolic BP ≥90 mmHg (Korotkoff V) on at least 2 occasions 4 h apart after 20 weeks' gestation, but before the onset of labour or postpartum systolic BP ≥ 140 mmHg and/or diastolic BP ≥90 mmHg on at least 2 occasions 4 h apart with any of the following new-onset conditions:
- proteinuria (≥ 300 mg/ 24 h or spot urine protein:creatinine ratio ≥ 30 mg/mmol creatinine, or urine dipstick protein > = ++).
- Other maternal organ dysfunction, including: Acute kidney Injury (creatinine >= 90 micromol / L or >= 1 mg/dL); Liver involvement (elevated transaminases, eg, alanine aminotransferase or aspartate aminotransferase >40 IU/L) with or without right upper quadrant or epigastric abdominal pain; Neurological complications (examples include eclampsia, altered mental status, blindness, stroke, clonus, severe headaches, and persistent visual scotomata); Hematological complications (thrombocytopenia-platelet count; <150 000/µL, disseminated intravascular coagulation, hemolysis)
- Or Uteroplacental dysfunction (such as fetal growth restriction, abnormal umbilical artery [UA] Doppler wave form analysis, or stillbirth) [13].

As used herein, the term "pre-term pre-eclampsia" should be understood to mean, a pre-eclampsia diagnosis which is made pre-term, e.g. before (<) 37 weeks of gestation and which warrants for the pre-term delivery of the baby, e.g. before (<) 37 weeks of gestation. As used herein, the term "term pre-eclampsia" should be understood to mean, a pre-eclampsia diagnosis which is made and whereby for delivery of the baby is term, e.g. at or after (≥) 37 weeks of gestation.

As used herein, the term "BMI" or "body mass index" as applied to a pregnant woman should be understood to mean the woman's weight (kg) divided by the square of the body height (m²) taken early in pregnancy, e.g. at 8-24 weeks pregnancy.

As used herein, the term "reference BMI" should be understood to mean an average BMI for pregnant women in a given population. For European women, this would be 25.

As used herein, the term "blood pressure" may be maternal diastolic or maternal systolic blood pressure, or mean arterial pressure (MAP) which is estimated as follows: MAP = [2/3 (diastolic bp) + 1/3 (systolic bp)].

As used herein, the term "reference blood pressure" should be understood to mean an average blood pressure for pregnant women in a given population. The methods and systems of the invention may optionally use a reference blood pressure for all pregnant women, a sub-population of pregnant women, or a reference blood pressure for high BMI pregnant women (i.e. when the pregnant woman is determined to have a high BMI) or a reference blood pressure for low BMI pregnant women (i.e. when the pregnant woman is determined to have a low BMI).

As used herein, the term "fetal sex" refers to the sex of the fetus carried by the pregnant woman. Fetal sex is generally determined early in pregnancy, at or around the same time as the BMI and blood pressure is determined. Fetal sex can be established by ultrasound. Ultrasound has been the traditional method used for fetal sex determination. In the second and third trimesters, it is accurate in >99% of cases with normal genitalia. Early ultrasound (12-14 weeks) is also a reliable option when performed at specialized centers. Invasive testing, either using chorionic villus sampling from 11 weeks or amniocentesis from 15 weeks is also an option. More preferably, fetal sex can be determined by fetal sex determination using cell-free fetal DNA as available in the maternal blood stream during pregnancy, for example using, but not limited to, DNA sequencing or PCR technology. In one embodiment, the fetal sex is determined by an in-vitro diagnostic method.

As used herein, the term "pre-eclampsia drug" refers to a therapeutic intervention for pregnant women to prevent development of pre-eclampsia typically during the second or third trimester of pregnancy. An Example of therapeutic intervention for preterm pre-eclampsia includes Aspirin. Examples of therapeutic intervention for term pre-eclampsia and / or preeclampsia in the high bmi group include: Low Molecular Weight Heparin; Restriction of weight gain by either caloric intake reduction or life style changes; Interventions to lower the glycemic index, including but not restricted to, insulin, glycemic index lowering probiotics; Citrulline; Antioxidants, including but not limited to, antioxidant vitamins (e.g., ascorbic acid, -tocopherol, -carotene), inorganic antioxidants (e.g., selenium), and a plant-derived polyphenols, antioxidants to mitochondria, including but not limited to, Mito VitE and ergothioneine; statins, including but not limited to, Pravastin. Therapies involving the use of anti-inflammatory or immunosuppressive agents like, but not limited to, tacrolimus, or sulfalazine. In addition, one can easily envision preferred therapeutic combinations like, but not limited to, aspirin and metformin; or metformin and sulfalazine.

As used herein, the term "metformin treatment" refers to treatment with Metformin (GLUCOPHAGE) or a combination therapy comprising Metformin and an addition drug, for example aspirin, thiazolidinediones, DPP-4 inhibitors, sulfonylureas, and meglitinide.

The embodiments described with reference to the drawings comprise a computer apparatus and/or processes performed in a computer apparatus. However, the invention also extends to computer programs, particularly computer programs stored on or in a carrier adapted to bring the invention into practice. The program may be in the form of source code, object code, or a code intermediate source and object code, such as in partially compiled form or in any other form suitable for use in the implementation of the method according to the invention. The carrier may comprise a storage medium such as ROM, e.g. CD ROM, or magnetic recording medium, e.g. a floppy disk or hard disk. The carrier may be an electrical or optical signal which may be transmitted via an electrical or an optical cable or by radio or other means.

### Exemplification

The invention will now be described with reference to specific Examples. These are merely exemplary and for illustrative purposes only: they are not intended to be limiting in any way to the scope of the invention, which is solely defined by the appended claims.

In a cohort of case-control study constituting 1336 pregnant women, blood pressure measurements were taken early in pregnancy. Within this group, 123 women developed preeclampsia and 1213 did not. The 1231 who did not develop preeclampsia were a representative cross-section of all other pregnancies: other complications of pregnancy like spontaneous preterm birth, gestational diabetes, fetal growth restriction etc... occurred in the control group. From the 123 women who developed preeclampsia, 32 developed preterm preeclampsia and 91 did develop term preeclampsia. The pregnancy population considered was deemed "low risk" for developing preeclampsia from a clinical perspective: the following women were excluded from the study: women with essential hypertension treated pre-pregnancy, women who had moderate-severe hypertension at booking (BP >160/100 mmHg), i.e., the first time they were seen in their pregnancies; women with diabetes, women with renal disease, women with systemic Lupus Erythematosus. Women with Anti-phospholipid syndrome, women with sickle cell disease, women who were treated with low-dose aspirin or heparin/low molecular weight heparin, women with a previous pregnancy⁴.

Other characteristics of the study population are summarised in below **Table 1:**

| | | Controls (Non-PE) | PE | Preterm PE | Term PE |
|---|---|---|---|---|---|
| **Number** | | N=1213 | N=123 | N=32 | N=91 |
| **Maternal Age, Yrs** | | | | | |
| | Mean (SD) | 29.591 (4.499) | 29.837 (4.866) | 30.343 (5.147) | 29.6593 (4.780) |
| | median (Q1,Q3) | 30.00 (27.00, 33.00) | 30.00 (26.00, 33.00) | 30 (27.00,32.25) | 30 (26.00, 33.00) |

| **BMI** | | | | | |
|---|---|---|---|---|---|
| | missing data (n) | 2 | | | |
| | Mean (SD) | 24.879 (4.116) | 26.486 (4.660) | 26.334 (3.530) | 26.540 (5.014) |
| | median (Q1,Q3) | 24.000 (22.000, 26.900) | 25.700 (22.900, 29.700) | 26.450 (23.450, 28.475) | 25.500 (22.750, 29.700) |
| | bmi<25 (n, (%)) / bmi>=25 (n, (%)) | 733 (60.53) / 478 (39.47%) | 55 (44.72%) / 68 (55.28) | 13 (40.63%) / 19 (59.38) | 42 (46.15%) / 49 (53.85%) |
| | bmi<30 (n, (%)) / bmi>=30 (n, (%)) | 1076 (88.85%) / 135 (11.15%) | 91 (78.86%)/26 (21.14%) | 26 (81.25%) / 6 (18.75%) | 71 (78.02%) / 20 (21.98%) |

| **Fetal Sex** | | | | | |
|---|---|---|---|---|---|
| | data unavailable (n, (%)) | 16 (1.32%) | | | |
| | Female Fetus (n, (%)) | 563 (46.41%) | 56 (45.53%) | 12 (37.5%) | 44 (48.35%) |
| | Male Fetus (n, (%)) | 634 (52.27%) | 67 (54.47%) | 20 (62.5%) | 47 (51.65%) |

| **2^{nd} MAP (mm Hg)** | | | | | |
|---|---|---|---|---|---|
| | mean (SD) | 83.719 (7.862) | 89.509 (8.912) | 90.521 (8.352) | 89.154 (9.118) |
| | median (Q1,Q3) | 83.000 (78.333, 88.667) | 89.000 (83.667, 95.167) | 88.83 (85.250, 95.750) | 89.000 (83.500, 95.000) |

| **Delivery (weeks)** | | | | | |
|---|---|---|---|---|---|
| | Mean (SD) | 39.837 (2.006) | 37.780 (3.399) | 33.103 (3.228) | 39.425 (1.247) |
| | median (Q1,Q3) | 40.286 (39.143, 41.000) | 38.857 (36.857, 40.00) | 34.00 (31.857, 35.857) | 39.429 (38.429, 40.429) |
| | Preterm Delivery (n,((%) | 61 (5.03%) | 32 (26.02%) | 32 (100%) | 0 (0%) |

Preeclampsia was diagnosed as follows: Preeclampsia (PE) was defined as gestational hypertension (systolic BP ≥ 140 mmHg and/or diastolic BP ≥90 mmHg (Korotkoff V) on at least 2 occasions 4 h apart after 20 weeks' gestation, but before the onset of labour or postpartum systolic BP ≥ 140 mmHg and/or diastolic BP ≥90 mmHg on at least 2 occasions 4 h apart with proteinuria (≥ 300 mg/ 24 h or spot urine protein:creatinine ratio ≥ 30 mg/mmol creatinine, or urine dipstick protein > = ++)

At about 15 weeks of gestation (14 + 0 to 16 + 6 weeks'), the following maternal measurements were performed: weight, height, blood pressure and entered in a database. A pregnant woman's status at the time of delivery was also recorded in a database. Information about pregnancy complications will also be recorded. All participants who developed pre-eclampsia had detailed clinical, laboratory and outcome data collected. All women consented to be part of this study.⁴

Fetal sex can be established by ultrasound. Ultrasound has been the traditional method used for fetal sex determination. In the second and third trimesters, it is accurate in >99% of cases with normal genitalia.⁵ Early ultrasound (12-14 weeks) is also a reliable option when performed at specialized centers.⁶ Invasive testing, either using chorionic villus sampling from 11 weeks or amniocentesis from 15 weeks is also an option^{7,8}. More preferably, fetal sex can be determined by fetal sex determination using cell-free fetal DNA as available in the maternal blood stream during pregnancy, for example using, but not limited to, DNA sequencing or PCR technology^{9,10}.

The discriminative performance of Mean Arterial Pressure (MAP) was quantified using the area under the receiver operating curve (AUROC) ¹¹. An AUROC of 0.5 or lower indicates an absence of predictive power for the outcome. The exact Binomial Confidence Interval for the Area Under the Curve was calculated. Mean Arterial Pressure is considered a predictor if its AUROC was significantly higher than 0.5 (p<0.05). The Mean Arterial Pressure as calculated from the second set of blood pressure measurements taken is used. In total there were three set of blood pressure measurements taken, each consisting out of a diastolic and systolic reading. Mean Arterial Pressures as calculated from the 1^{st}, 2^{nd} and 3^{rd} readings did not differ significantly; the Mean Arterial Pressure as calculated from the 2^{nd} set of blood pressure readings was therefore arbitrarily taken for reporting.

The added value of refining pre-eclampsia prediction by sub-typing the pregnancy in terms of the maternal characteristic "BMI", or/and the fetal characteristic "fetal sex" and sub-typing the pre-eclampsia outcome in terms of gestational age at pre-eclampsia indicated delivery was quantified by calculating the absolute difference in AUROC for preeclampsia prediction in the sub-types created, whereby the following criteria were applied
- Absolute Delta AUROC >=0.1: distinct added value of sub-typing (bold in tables)
- 0.1 > Absolute Delta AUROC >=0.05: some added value of sub-typing (*Italic* in tables)

From the following table 2 (Figure 1), it is clear that the classic risk predictor blood pressure (MAP) has some predictive performance for predicting preeclampsia in this population [AUROC = 0.69]; as well known. When assessing the predictive performance of MAP to predict either outcome sub-types *preterm PE* or *term PE* in the whole population, the predictive performance remains largely the same; only some minor prediction refinement is achieved (cf. Delta AUROC = 0.05)

However, when one uses the height (in meter) and weight (in kg) to calculate the pregnant woman's BMI, and uses this information to classify women in either a low BMI group or a high BMI group, blood pressure allows to differentially predict preterm PE and term PE. It is found that MAP (as a representative blood pressure measurement) has exceptional preterm PE prediction performance in the low BMI group [AUROC > 0.80], yet no meaningful predictive performance for preterm PE in the high BMI group. Conversely, MAP is a poor predictor for term PE in the low BMI group and an average predictor for term PE in the high BMI group [AUROC > 0.70]; Table 3 and Appendix 1 - Figures 2 and 3.

Alternatively, when one takes into consideration the fetal characteristic, fetal sex (Female or Male), the refinement of preeclampsia risk prediction for preeclampsia or the preeclampsia outcome subtypes preterm preeclampsia or term preeclampsia, is either negligible (Prediction of PE and Prediction of term PE) or minor. For preterm preeclampsia, incorporating fetal sex information delivers some minor prediction refinement (cf. Delta AUROC = 0.079), with improved preterm preeclampsia prediction for women carrying a female fetus [AUC > 0.75]. See also table 4 and Appendix 1 - Figures 4 and 5.

However, when one uses the height (in meter) and weight (in kg) to calculate the pregnant woman's BMI, uses this information to classify women in either a low BMI group or a high BMI group, and then further stratify the women in function of the sex of the fetus they carry, prediction of preeclampsia and its subtypes preterm preeclampsia and term preeclampsia using blood pressure can be further refined.

It is found that the exceptional preterm PE prediction performance in the low BMI group of MAP (as a representative blood pressure measurement) is accentuated further in women carrying a female fetus [AUROC > 0.9] compared to women carrying a male fetus [AUROC < 0.80]. The lack of preterm preeclampsia prediction using MAP is maintained in the high BMI group irrespective of using fetal sex information.

Whereas MAP appears a poor predictor for term PE in the low BMI group [AUROC = 0.615], additional sub-typing based on fetal sex information reveals that MAP does have some prediction performance for term PE in women carrying a female fetus [AUROC > 0.65] compared to women carrying a male fetus [AUROC < 0.60]. Conversely, in the women in the high BMI group, additional sub-typing based on fetal sex improves prediction of term preeclampsia carrying a male fetus [AUROC > 0.75] compared to women carrying a female fetus [AUROC < 0.70]; Table 5 and Appendix 1 - Figures 6 and 7

**Table 2**

| | | **controls (non PE)** | | **PE Cases** | | **Prediction (AUROC)** | | | |
|---|---|---|---|---|---|---|---|---|---|
| | | **n** | **Mean MAP (SD)** | **n** | **Mean MAP (SD)** | **AUROC** | **AUROC 95%CI** | **p-value** | |
| **Prediction of PE** | ***All Pregnancies*** | 1213 | 83.719 (7.862) | 123 | 89.509 (8.912) | 0.691 | 0.665 - 0.716 | <0.001 | Δ AUROC |
| **Prediction of preterm PE** | ***All Pregnancies*** | 1213 | 83.719 (7.862) | 32 | 90.521 (8.352) | 0.726 | 0.700 - 0.750 | *<0.001* | *0.055* |
| **Prediction of term PE** | ***All Pregnancies*** | 1213 | 83.719 (7.862) | 91 | 89.154 (9.118) | 0.671 | 0.652 - 0.704 | *<0.001* | |

**Table 3**

| | | | **controls (non PE)** | | **PE Cases** | | **Prediction (AUROC)** | | | |
|---|---|---|---|---|---|---|---|---|---|---|
| | | | **n** | **Mean MAP (SD)** | **n** | **Mean MAP (SD)** | **AUROC** | **AUROC 95%CI** | **p-value** | |
| **Prediction of PE** | ***All Pregnancies*** | | 1213 | 83.719 (7.862) | 123 | 89.509 (8.912) | 0.691 | 0.665 - 0.716 | <0.001 | Δ AUROC |
| | | *Pregnancies BMI <25* | 733 | 81.819 (7.457) | 55 | 86.297 (7.785) | 0.667 | 0.632 - 0.699 | <0.001 | 0.016 |
| | | *Pregnancies BMI >=25* | 478 | 86.614 (7.584) | 68 | 92.108 ( 8.969) | 0.683 | 0.642 - 0.722 | <0.001 | |
| **Prediction of preterm PE** | ***All Pregnancies*** | | 1213 | 83.719 (7.862) | 32 | 90.521 (8.352) | 0.726 | 0.700 - 0.750 | <0.001 | Δ AUROC |
| | | *Pregnancies BMI <25* | 733 | 81.819 (7.457) | 13 | 90.462 (4.879) | **0.834** | 0.806 - 0.860 | <0.001 | **0.238** |
| | | *Pregnancies BMI >=25* | 478 | 86.614 (7.584) | 19 | 90.561 (10.218) | 0.596 | 0.551 - 0.639 | 0.18 | |
| Prediction of term **PE** | ***All Pregnancies*** | | 1213 | 83.719 (7.862) | 91 | 89.154 (9.118) | 0.671 | 0.652 - 0.704 | <0.001 | Δ AUROC |
| | | *Pregnancies BMI <25* | 733 | 81.819 (7.457) | 42 | 85.008 (8.103) | 0.615 | 0.579 - 0.649 | 0.014 | **0.102** |
| | | *Pregnancies BMI >=25* | 478 | 86.614 (7.584) | 49 | 92.707 (8.477) | **0.717** | 0.677 - 0.755 | <0.001 | |

**Table 4**

| | | | **controls (non PE)** | | **PE Cases** | | **Prediction (AUROC)** | | | |
|---|---|---|---|---|---|---|---|---|---|---|
| | | | **n** | **Mean MAP (SD)** | **n** | **Mean MAP (SD)** | **AUROC** | **AUROC 95%CI** | **p-value** | |
| Prediction of **PE** | ***All Pregnancies*** | | 1213 | 83.719 (7.862) | 123 | 89.509 (8.912) | 0.691 | 0.665 - 0.716 | <0.001 | Δ AUROC |
| | | *& female fetus* | 563 | 83.355 (7.665) | 56 | 88.118 (8.904) | 0.698 | 0.660 - 0.734 | <0.001 | 0.013 |
| | | *& male fetus* | 634 | 84.028 (8.048) | 67 | 89.836 (8.972) | 0.685 | 0.649 - 0.719 | <0.001 | |
| **Prediction of preterm PE** | ***All Pregnancies*** | | 1213 | 83.719 (7.862) | 32 | 90.521 (8.352) | 0.726 | 0.700 - 0.750 | <0.001 | Δ AUROC |
| | | *& female fetus* | 563 | 83.355 (7.665) | 12 | 91.028 (7.213) | 0.773 | 0.737 - 0.807 | <0.001 | *0.079* |
| | | *& male fetus* | 634 | 84.028 (8.048) | 20 | 90.217 (9.134) | 0.694 | 0.657 - 0.729 | <0.001 | |
| **Prediction of term PE** | ***All Pregnancies*** | | 1213 | 83.719 (7.862) | 91 | 89.154 (9.118) | 0.671 | 0.652 - 0.704 | <0.001 | Δ AUROC |
| | | *& female fetus* | 563 | 83.355 (7.665) | 44 | 88.598 (9.316) | 0.678 | 0.639 - 0.715 | <0.001 | 0.004 |
| | | *& male fetus* | 634 | 84.028 (8.048) | 47 | 89.674 (8.997) | 0.682 | 0.645 - 0.716 | <0.001 | |

**Table 5**

| | | | **controls (non PE)** | | **PE Cases** | | **Prediction (AUROC)** | | | |
|---|---|---|---|---|---|---|---|---|---|---|
| | | | **n** | **Mean MAP (SD)** | **n** | **Mean MAP (SD)** | **AUROC** | **AUROC 95%CI** | **p-value** | |
| **Prediction of PE** | ***All Pregnancies*** | | 1213 | 83.719 (7.862) | 123 | 89.509 (8.912) | 0.691 | 0.665 - 0.716 | <0.001 | Δ AUROC |
| | | *& female fetus* | 563 | 83.355 (7.665) | 56 | 88.118 (8.904) | 0.698 | 0.660 - 0.734 | <0.001 | 0.013 |
| | | *& male fetus* | 634 | 84.028 (8.048) | 67 | 89.836 (8.972) | 0.685 | 0.649 - 0.719 | <0.001 | |
| | ***Pregnancies BMI <25*** | | 733 | 81.819 (7.457) | 55 | 86.297 (7.785) | 0.667 | 0.632 - 0.699 | <0.001 | Δ AUROC |
| | | *& female fetus* | 334 | 81.300 (7.112) | 25 | 87.347 (8.431) | 0.713 | 0.663 - 0.759 | <0.001 | *0.086* |
| | | *& male fetus* | 391 | 82.194 (7.692) | 30 | 85.167 (7.231) | 0.627 | 0.578 - 0.673 | <0.001 | |
| | ***Pregnancies BMI >=25*** | | 478 | 86.614 (7.584) | 68 | 92.108 ( 8.969) | 0.683 | 0.642 - 0.722 | <0.001 | Δ AUROC |
| | | *& female fetus* | 228 | 86.349 (7.483) | 31 | 90.548 (8.151) | 0.643 | 0.581 - 0.701 | 0.011 | *0.068* |
| | | *& male fetus* | 243 | 86.978 (7.739) | 37 | 93.414 (8.722) | 0.711 | 0.654 - 0.763 | <0.001 | |
| Prediction of preterm **PE** | ***All Pregnancies*** | | 1213 | 83.719 (7.862) | 32 | 90.521 (8.352) | 0.726 | 0.700 - 0.750 | <0.001 | Δ AUROC |
| | | *& female fetus* | 563 | 83.355 (7.665) | 12 | 91.028 (7.213) | 0.773 | 0.737 - 0.807 | <0.001 | *0.079* |
| | | *& male fetus* | 634 | 84.028 (8.048) | 20 | 90.217 (9.134) | 0.694 | 0.657 - 0.729 | <0.001 | |
| | ***Pregnancies BMI <25*** | | 733 | 81.819 (7.457) | 13 | 90.462 (4.879) | 0.834 | 0.806 - 0.860 | <0.001 | Δ AUROC |
| | | *& female fetus* | 334 | 81.300 (7.112) | 4 | 93.583 (3.604) | **0.94** | 0.901 - 0.963 | <0.001 | **0.167** |
| | | *& male fetus* | 391 | 82.194 (7.692) | 9 | 89.074 (4.879) | 0.773 | 0.729 -0.813 | <0.001 | |
| | ***Pregnancies BMI >=25*** | | 478 | 86.614 (7.584) | 19 | 90.561 (10.218) | 0.596 | 0.551 - 0.639 | 0.18 | Δ AUROC |
| | | *& female fetus* | 228 | 86.349 (7.483) | 8 | 89.750 (8.402) | 0.602 | 0.536 - 0.665 | *0.36* | 0.014 |
| | | *& male fetus* | 243 | 86.978 (7.739) | 11 | 91.152 (11.719) | 0.588 | 0.523 - 0.649 | 0.38 | |
| Prediction of term **PE** | ***All Pregnancies*** | | 1213 | 83.719 (7.862) | 91 | 89.154 (9.118) | 0.671 | 0.652 - 0.704 | <0.001 | Δ AUROC |
| | | *& female fetus* | 563 | 83.355 (7.665) | 44 | 88.598 (9.316) | 0.678 | 0.639 - 0.715 | <0.001 | 0.004 |
| | | *& male fetus* | 634 | 84.028 (8.048) | 47 | 89.674 (8.997) | 0.682 | 0.645 - 0.716 | <0.001 | |
| | ***Pregnancies BMI <25*** | | 733 | 81.819 (7.457) | 42 | 85.008 (8.103) | 0.615 | 0.579 - 0.649 | 0.014 | Δ AUROC |
| | | *& female fetus* | 334 | 81.300 (7.112) | 21 | 86.159 (8.608) | 0.67 | 0.618 - 0.719 | 0.017 | 0.106 |
| | | *& male fetus* | 391 | 82.194 (7.692) | 21 | 83.857 (7.597) | 0.564 | 0.514 - 0.612 | 0.298 | |
| | ***Pregnancies BMI >=25*** | | 478 | 86.614 (7.584) | 49 | 92.707 (8.477) | 0.717 | 0.677 - 0.755 | <0.001 | Δ AUROC |
| | | *& female fetus* | 228 | 86.349 (7.483) | 23 | 90.826 (9.561) | 0.657 | 0.595 - 0.712 | 0.014 | 0.106 |
| | | *& male fetus* | 243 | 86.978 (7.739) | 26 | 94.372 (7.169) | **0.763** | 0.708 - 0.812 | <0.001 | |

### Equivalents

The foregoing description details presently preferred embodiments of the present invention.

It is understood that the scope of the invention is solely defined by the appended claims.

### References:

1. Steegers, E. a P., von Dadelszen, P., Duvekot, J. J. & Pijnenborg, R. Pre-eclampsia. Lancet 376, 631-44 (2010).
2. Wright, D. et al. Aspirin for Evidence-Based Preeclampsia Prevention trial: effect of aspirin on length of stay in the neonatal intensive care unit. Am. J. Obstet. Gynecol. 218, 612.e1-612.e6 (2018).
3. Syngelaki, A. et al. Metformin versus Placebo in Obese Pregnant Women without Diabetes Mellitus. N. Engl. J. Med. 374, 434-43 (2016).
4. Navaratnam, K. et al. A multi-centre phase IIa clinical study of predictive testing for preeclampsia: improved pregnancy outcomes via early detection (IMPROvED). BMC Pregnancy Childbirth 13, 226 (2013).
5. Emerson, D. S., Felker, R. E. & Brown, D. L. The sagittal sign. An early second trimester sonographic indicator of fetal gender. J. Ultrasound Med. 8, 293-297 (1989).
6. Efrat, Z., Akinfenwa, O. O. & Nicolaides, K. H. First-trimester determination of fetal gender by ultrasound. Ultrasound Obstet. Gynecol. 13, 305-307 (1999).
7. Alfirevic, Z., Mujezinovic, F. & Sundberg, K. Amniocentesis and chorionic villus sampling for prenatal diagnosis. in Cochrane Database of Systematic Reviews (ed. Alfirevic, Z.) 148-156 (John Wiley & Sons, Ltd, 2003). doi:10.1002/14651858.CD003252
8. Hackett, G. A. et al. Early amniocentesis at 11-14 weeks' gestation for the diagnosis of fetal chromosomal abnormality-a clinical evaluation. Prenat. Diagn. 11, 311-315 (1991).
9. Mazloom, A. R. et al. Noninvasive prenatal detection of sex chromosomal aneuploidies by sequencing circulating cell-free DNA from maternal plasma. Prenat. Diagn. 33, 591-597 (2013).
10. Costa, J. M. et al. First-trimester fetal sex determination in maternal serum using real-time PCR. Prenat. Diagn. 21, 1070-4 (2001).
11. Zweig, M. H. & Campbell, G. Receiver-operating characteristic (ROC) plots: a fundamental evaluation tool in clinical medicine. Clin. Chem. 39, 561-77 (1993).
12. Catalano, P. M. & Shankar, K. Obesity and pregnancy: Mechanisms of short term and long term adverse consequences for mother and child. BMJ 356, 1-37 (2017).
13. Brown et al (Hypertensive disorders of Pregnancy, Hypertension, 2018; 72: 24-43).

## Claims

1. A system to determine risk of a pregnant woman subsequently developing preterm or term preeclampsia, comprising a computer processor configured to:
receive as inputs the blood pressure and body mass index, BMI, of the pregnant woman at 13 to 19 weeks of pregnancy;
compare the BMI of the pregnant woman with a reference BMI to determine if the BMI of the pregnant woman is higher or lower than a reference BMI;
when the BMI is determined to be higher than a reference BMI, compare the blood pressure of the pregnant woman with a reference blood pressure for a high BMI pregnant woman, or when the BMI is determined to be lower than a reference BMI, compare the blood pressure of the pregnant woman with a reference blood pressure for a low BMI pregnant woman; and
output a risk of term or pre-term pre-eclampsia based on the comparison,
wherein:
when the woman exhibits blood pressure higher than the reference blood pressure and a BMI lower than the reference BMI, the output is a prediction of an elevated risk of the pregnant woman subsequently developing **pre-term** pre-eclampsia;
when the woman exhibits blood pressure lower than the reference blood pressure and a BMI lower than the reference BMI, the output is a prediction that the pregnant woman is not at elevated risk of subsequently developing **pre-term** pre-eclampsia;
when the woman exhibits blood pressure higher than the reference blood pressure and a BMI higher than the reference BMI, the output is a prediction of an elevated risk of the pregnant woman subsequently developing **term** pre-eclampsia; or
when the woman exhibits blood pressure lower than the reference blood pressure and a BMI higher than the reference BMI, the output is a prediction that the pregnant woman is not at elevated risk of subsequently developing **term** pre-eclampsia.

2. A system according to Claim 1, in which the computer processor is configured to receive the height and weight of the pregnant woman and calculate the BMI of the pregnant woman.

3. A system according to any of Claims 1 or 2, in which the computer processor is configured to
receive as an additional input the fetal sex,
in which:
when the woman exhibits blood pressure higher than the reference blood pressure, a BMI lower than the reference BMI, and the fetal sex is female, the output is a prediction of elevated risk of the pregnant woman subsequently developing pre-term pre-eclampsia; or
when the woman exhibits blood pressure higher than the reference blood pressure, a BMI higher than the reference BMI, and the fetal sex is male, the output is a prediction of elevated risk of the pregnant woman subsequently developing term pre-eclampsia.

4. A system according to any preceding Claim, comprising a blood pressure measurement apparatus, height measurement apparatus, and weight measurement apparatus operatively coupled to the computer processor.

5. A system according to any preceding Claim in which the risk of term or pre-term pre-eclampsia is based completely on a comparison of the BMI with a reference BMI and the blood pressure input with a reference blood pressure, and optionally the fetal sex.

## Patentansprüche

1. System zur Bestimmung eines Risikos, dass eine schwangere Frau im Folgenden Früh- oder Terminpräeklampsie entwickelt, das einen Computerprozessor umfasst, der für Folgendes konfiguriert ist:
Empfangen des Blutdrucks und Body-Mass-Index, BMI, der schwangeren Frau bei 13 bis 19 Wochen der Schwangerschaft als Eingaben;
Vergleichen des BMI der schwangeren Frau mit einem Referenz-BMI zur Bestimmung, ob der BMI der schwangeren Frau höher oder niedriger ist als ein Referenz-BMI;
bei Bestimmung, dass der BMI höher ist als ein Referenz-BMI, Vergleichen des Blutdrucks der schwangeren Frau mit einem Referenzblutdruck für eine schwangere Frau mit hohem BMI oder bei Bestimmung, dass der BMI niedriger ist als ein Referenz-BMI, Vergleichen des Blutdrucks der schwangeren Frau mit einem Referenzblutdruck für eine schwangere Frau mit niedrigem BMI; und
Ausgabe eines Risikos von Termin- oder Frühpräeklampsie basierend auf dem Vergleich,
wobei:
wenn die Frau einen Blutdruck hat, der höher ist als der Referenzblutdruck, und einen BMI hat, der niedriger ist als der Referenz-BMI, die Ausgabe eine Vorhersage eines erhöhten Risikos ist, dass die schwangere Frau im Folgenden Frühpräeklampsie entwickelt;
wenn die Frau einen Blutdruck hat, der niedriger ist als der Referenzblutdruck, und einen BMI hat, der niedriger ist als der Referenz-BMI, die Ausgabe eine Vorhersage ist, dass bei der schwangeren Frau kein erhöhtes Risiko besteht, dass sie im Folgenden Frühpräeklampsie entwickelt;
wenn die Frau einen Blutdruck hat, der höher ist als der Referenzblutdruck, und einen BMI hat, der höher ist als der Referenz-BMI, die Ausgabe eine Vorhersage eines erhöhten Risikos ist, dass die schwangere Frau im Folgenden Terminpräeklampsie entwickelt; oder
wenn die Frau einen Blutdruck hat, der niedriger ist als der Referenzblutdruck, und einen BMI hat, der höher ist als der Referenz-BMI, die Ausgabe eine Vorhersage ist, dass bei der schwangeren Frau kein erhöhtes Risiko besteht, dass sie im Folgenden Terminpräeklampsie entwickelt.

2. System nach Anspruch 1, in dem der Computerprozessor zum Empfang der Größe und des Gewichts der schwangeren Frau und zur Berechnung des BMI der schwangeren Frau konfiguriert ist.

3. System nach einem der Ansprüche 1 oder 2, in dem der Computerprozessor für Folgendes konfiguriert ist:
Empfangen des fetalen Geschlechts als eine zusätzliche Eingabe,
wobei:
wenn die Frau einen Blutdruck hat, der höher ist als der Referenzblutdruck, einen BMI hat, der niedriger ist als der Referenz-BMI, und das fetale Geschlecht weiblich ist, die Ausgabe eine Vorhersage eines erhöhten Risikos ist, dass die schwangere Frau im Folgenden Frühpräeklampsie entwickelt; oder
wenn die Frau einen Blutdruck hat, der höher ist als der Referenzblutdruck, einen BMI hat, der höher ist als der Referenz-BMI, und das fetale Geschlecht männlich ist, die Ausgabe eine Vorhersage eines erhöhten Risikos ist, dass die schwangere Frau im Folgenden Terminpräeklampsie entwickelt.

4. System nach einem vorstehenden Anspruch, das ein Blutdruckmessgerät, Größenmessgerät und Gewichtsmessgerät umfasst, die funktionsfähig mit dem Computerprozessor verbunden sind.

5. System nach einem vorstehenden Anspruch, in dem das Risiko von Termin- oder Frühpräeklampsie vollständig auf einem Vergleich des BMI mit einem Referenz-BMI und der Blutdruckeingabe mit einem Referenzblutdruck und optional dem fetalen Geschlecht basiert.

## Revendications

1. Système pour déterminer un risque qu'une femme enceinte développe ultérieurement une pré-éclampsie pré-terme ou à terme, comprenant un processeur informatique configuré pour :
recevoir comme entrées la pression artérielle et l'indice de masse corporelle, IMC, de la femme enceinte à 13 à 19 semaines de grossesse ;
comparer l'IMC de la femme enceinte à un IMC de référence pour déterminer si l'IMC de la femme enceinte est supérieur ou inférieur à un IMC de référence ;
lorsqu'il est déterminé que l'IMC est supérieur à un IMC de référence, comparer la pression artérielle de la femme enceinte à une pression artérielle de référence pour une femme enceinte ayant un IMC élevé, ou lorsqu'il est déterminé que l'IMC est inférieur à un IMC de référence, comparer la pression artérielle de la femme enceinte à une pression artérielle de référence pour une femme enceinte ayant un IMC faible ; et
délivrer en sortie un risque de pré-éclampsie à terme ou pré-terme sur la base de la comparaison, dans lequel :
lorsque la femme présente une pression artérielle supérieure à la pression artérielle de référence et un IMC inférieur à l'IMC de référence, la sortie est une prédiction d'un risque élevé que la femme enceinte développe ultérieurement une pré-éclampsie pré-terme ;
lorsque la femme présente une pression artérielle inférieure à la pression artérielle de référence et un IMC inférieur à l'IMC de référence, la sortie est une prédiction selon laquelle la femme enceinte n'est pas à risque élevé de développer ultérieurement une pré-éclampsie pré-terme ;
lorsque la femme présente une pression artérielle supérieure à la pression artérielle de référence et un IMC supérieur à l'IMC de référence, la sortie est une prédiction d'un risque élevé que la femme enceinte développe ultérieurement une pré-éclampsie à terme ; ou
lorsque la femme présente une pression artérielle inférieure à la pression artérielle de référence et un IMC supérieur à l'IMC de référence, la sortie est une prédiction selon laquelle la femme enceinte n'est pas à risque élevé de développer ultérieurement une pré-éclampsie à terme.

2. Système selon la revendication 1, dans lequel le processeur informatique est configuré pour recevoir la taille et le poids de la femme enceinte et calculer l'IMC de la femme enceinte.

3. Système selon l'une quelconque des revendications 1 ou 2, dans lequel le processeur informatique est configuré pour recevoir comme entrée supplémentaire le sexe fœtal, dans lequel :
lorsque la femme présente une pression artérielle supérieure à la pression artérielle de référence, un IMC inférieur à l'IMC de référence, et le sexe fœtal est féminin, la sortie est une prédiction d'un risque élevé que la femme enceinte développe ultérieurement une pré-éclampsie pré-terme ; ou
lorsque la femme présente une pression artérielle supérieure à la pression artérielle de référence, un IMC supérieur à l'IMC de référence, et le sexe fœtal est masculin, la sortie est une prédiction d'un risque élevé que la femme enceinte développe ultérieurement une pré-éclampsie à terme.

4. Système selon l'une quelconque des revendications précédentes, comprenant un appareil de mesure de pression artérielle, un appareil de mesure de taille, et un appareil de mesure de poids couplés fonctionnellement au processeur informatique.

5. Système selon l'une quelconque des revendications précédentes, dans lequel le risque de pré-éclampsie à terme ou pré-terme est basé complètement sur une comparaison de l'IMC avec un IMC de référence et de l'entrée de pression artérielle avec une pression artérielle de référence, et éventuellement le sexe fœtal.
